(19) Europäisches Patentamt
European Patent Office
Office européen des brevets

(11) Veröffentlichungsnummer: **0 005 804**
**B1**

(12) # EUROPÄISCHE PATENTSCHRIFT

(21) Anmeldenummer: **79101602.5**

(22) Anmeldetag: **24.05.79**

(51) Int. Cl.[3]: **G 03 C 1/06,** C 07 C 149/24, C 07 C 149/26, C 07 C 149/437

(54) **Photographische Materialien, Verfahren zu ihrer Herstellung sowie zur Herstellung photographischer Bilder und neue Thioether**

(30) Priorität: **02.06.78 DE 2824249**

(43) Veröffentlichungstag der Anmeldung:
**12.12.79 Patentblatt 79/25**

(45) Bekanntmachung des Hinweises auf die Patenterteilung:
**26.11.80 Patentblatt 80/24**

(84) Benannte Vertragsstaaten:
**BE DE FR GB**

(56) Entgegenhaltungen:
**CH - A - 348 961**
**CH - A - 474 494**
**DE - B - 1 068 244**
**DE - A - 2 159 379**
**FR - A - 1 228 810**
**FR - A - 2 307 292**

(73) Patentinhaber: **AGFA-GEVAERT AKTIENGESELLSCHAFT**
**Patentabteilung**
**D - 5090 Leverkusen 1 (DE)**

(72) Erfinder: **Bergthaller, Peter, Dr.**
**Morgengraben 1**
**D - 5000 Köln 80 (DE)**
**Saleck, Wilhelm, Dr.**
**Im Birkelshof 3**
**D - 5060 Berg. Gladbach (DE)**
**Lapp, Otto, Dr.**
**Heymannstrasse 28**
**D - 5090 Leverkusen 1 (DE)**
**Mücke, Bruno, Dr.**
**Wipperfürther Strasse 105**
**D - 5060 Bergisch-Gladbach (DE)**

**EP 0 005 804 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Photographische Materialien, Verfahren zu ihrer Herstellung sowie zur Herstellung photographischer Bilder und neue Thioether

Die Erfindung betrifft neue Thioether und ihre Verwendung in photographischen Materialien. Die Erfindung betrifft insbesondere ein Verfahren zur Herstellung von photographischen Materialien mit mindestens einer Silberhalogenid-emulsion, bei dem die Fällung der Silberhalogenide durch Umsetzung eines wasserlöslichen Silbersalzes mit einem Alkalihalogenid in einem Bindemittelmedium in Gegenwart der erfindungsgemäßen Thioether ausgeführt wird.

Es sind verschiedene Verfahren bekannt, lichtempfindliche Silberhalogenidemulsionen herzustellen. Die Eigenschaften dieser Emulsionen werden vor allem bestimmt durch die Kristallgröße der Silberhalogenidkörner und deren Korngrößenverteilung. In speziellen Fällen spielt auch der Kristallhabitus eine entscheidende Rolle.

Die Größe der Silberhalogenidkristalle wird vor allem durch die sogenannte Ostwald-Reifung festgelegt, bei der größere Kristalle auf Kosten kleinerer Kristalle gleicher Zusammensetzung wachsen. Ein wichtiger Parameter bei der Ostwald-Reifung ist der Überschuß an Halogenidionen, die bekanntlich durch Komplexbildung einen Transport der Silberionen von den kleineren Kristallen zu den größeren Kristallen ermöglichen.

Es ist weiterhin bekkant, monodisperse Silberhalogenidemulsionen herzustellen, also Emulsionen mit einer außerordentlich engen Korngrößenverteilung. Bei der Herstellung derartiger monodisperser Emulsionen wird ein Kornwachstum, wie es bei der Ostwald-Reifung auftritt, vermieden. Mit Hilfe der bisher bekannten Verfahren gelingt es aber nicht in befriedigendem Umfang, die Anzahl der bei der Fällung entstehenden Silberhalogenidkeime derart zu begrenzen, daß von vornherein nur so viele Silberhalogenidkeime entstehen, wie Silberhalogenidkristalle benötigt werden.

Es ist bekannt, die Ostwald-Reifung bei der Herstellung heterodisperser Emulsion wie auch das Kristallwachstum bei der Herstellung monodisperser Emulsionen durch Zusatz von Thioethern zu beeinflussen. Beispielsweise wird in der deutschen Auslegeschrift 1 904 148 ein Verfahren zur Herstellung verschleierter direktpositiver Silberhalogenidemulsionen in Gegenwart bestimmter Thioether beschrieben. Die bekannten Thioether haben aber den Nachteil, daß sie in unerwünschter Weise einen Schleier verursachen können oder daß sie nur in sehr großen Mengen wirksam sind. Eine Aufgabe der Erfindung ist daher, neue Thioether aufzufinden, die die Nachteile bekannter Thioether nicht aufweisen.

Es wurden nun neue Thioether der folgenden Formel I aufgefunden:

$$\text{I} \qquad R^1\!-\!S\!-\!\underset{R^3}{\overset{R^2}{C}}\!-\!\underset{R^5}{\overset{R^4}{C}}\!-\!S\!-\!(CR^6R^7)_n\!-\!NR^8\!-\!Ac$$

worin bedeuten:

$R^1$ Aliphatischer, cycloaliphatischer, Aryl- oder Aralkyl-Rest;
$R^2$, $R^3$, $R^4$, $R^5$, $R^6$, $R^7$ gleich oder verschieden, Wasserstoff oder Alkyl;
$R^8$ Wasserstoff;
Ac Acrylrest;
n eine ganze Zahl, mindestens 1.

Die Eigenschaften photographischer Materialien werden durch Verwendung der Thioether gemäß Formel I deutlich verbessert.

Weiterhin wurde ein Verfahren zur Herstellung photographischer Materialien mit wenigstens einer Silberhalogenidemulsionsschicht gefunden, bei dem die Silberhalogenidemulsion in Gegenwart wenigstens eines Thioethers der Formel I hergestellt wird.

Weiterhin wurde ein photographisches Material mit wenigstens einer Silberhalogenidemulsionsschicht gefunden, das einen Thioether der Formel I enthält.

Weiterhin wurde ein Verfahren zur Herstellung photographischer Bilder durch bildmäßiges Belichten eines photographischen Materials mit wenigstens einer licht-empfindlichen Silberhalogenidemulsionsschicht, Entwicklung und übliche Weiterverarbeitung gefunden, bei dem die Silberhalogenidkörner der Silberhalogenidemulsionsschicht in Gegenwart eines Thioethers der Formel I hergestellt worden sind.

Unter Acylresten werden insbesondere solche verstanden, die sich von aliphatischen oder aromatischen Dicarbonoder Sulfocarbonsäuren, Kohlensäuremonoestern und Carbaminsäuren ableiten. Beispiele für derartige Acylreste sind Succinoyl, Carbamoyl, Phenylcarbamoyl, Sulfonamidocarbonyl und Acylaminocarbonyl.

Vorzugsweise entsprechen die Thioether folgender Struktur II:

II 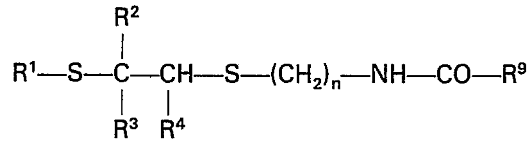

worin bedeuten:

$R^1$ aliphatischer oder cycloaliphatischer Rest mit maximal 6 C-Atomen oder Aralkyl mit maximal 8 C-Atomen;

$R^2$, $R^3$, $R^4$ gleich oder verschieden, Wasserstoff, Alkyl mit 1—7 C-Atomen, insbesondere Methyl;

n 2 oder 3;

$R^9$ —NH—$R^{10}$, —$R^{11}$—COOM oder —$R^{11}$—$SO_3M$;

M Metallion, insbesondere Alkali oder Erdalkalimetallion;

$R^{10}$ Wasserstoff, Alkyl mit 1—3 C-Atomen; ein Alkoxyalkyl-, Hydroxyalkyl-, Alkansulfonyl- oder Acylrest;

$R^{11}$ ein gegebenenfalls zusätzlich substituierter aliphatischer, cycloaliphatischer oder aromatischer Rest, der insbesondere mit einer Alkylthiogruppe substituiert sein kann

und/oder

$R^1$ zusammen mit $R^2$ und gegebenenfalls $R^3$ den zur Vervollständigung eines heterocyclischen Ringes, insbesondere eines 5- oder 6-gliedrigen Ringes, erforderlichen Rest und/oder

$R^4$ zusammen mit $R^2$ oder $R^3$ den zur Vervollständigung eines cycloaliphatischen, insbesondere eines 5- oder 6-gliedrigen Ringes, insbesondere eines Cyclohexanringes erforderlichen Rest.

Es wurde weiterhin gefunden, daß die Darstellung der Thioether der angegebenen Formel I in an sich bekannter Weise über die entsprechenden Amine der Formel III erfolgen kann:

III 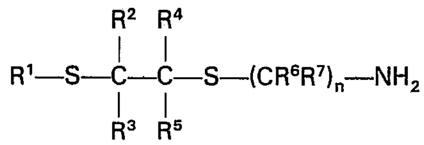

worin n und $R^1$, $R^2$, $R^3$, $R^4$, $R^5$, $R^6$ und $R^7$ die unter Formel I angegebene Bedeutung haben.

Hierzu setzt man die Amine der Formel III mit Alkalicyanaten, Oxazolidin-2-onen, Acylisocyanaten, Alkylisocyanaten, Alkansulfonylisocyanaten, Dicarbonsäureanhydriden oder Sulfocarbonsäureanhydriden um. Als Lösungsmittel für die Umsetzung mit Alkalicyanaten eignen sich insbesondere Wasser, Alkohole, Aceton und Acetonitril oder Gemische davon, wobei die Amine in Form leicht löslicher Salze, z.B. der Hydrochloride, Sulfate, Perchlorate, Nitrate oder Alkansulfonate, eingesetzt werden. Für die anderen Umsetzungen werden die Amine bevorzugt in Form der freien Base in vorzugsweise nicht-wässrigen Lösungen umgesetzt, beispielsweise in Ethylacetat, Dichlormethan, Acetonitril, 1,2-Dichlorethan oder Dimethylformamid.

Die Herstellung der der Formel III entsprechenden Amine erfolgt vorzugsweise ausgehend von Aminen folgender Formel IV:

IV 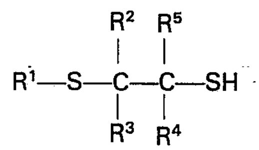

worin $R^1$, $R^2$, $R^3$, $R^4$ und $R^5$ die unter Formel I angegebene Bedeutung haben.

Derartige Umsetzungen sind beschrieben in der deutschen Offenlegungsschrift 1 904 149. Als Reaktionspartner dienen vorzugsweise Aziridin, Ammoniumethylsulfat, Chlorethylamin, Bromethylamin oder 3-Chlorpropylamin. Die Umsetzung erfolgt vorzugsweise im neutralen oder alkalischen Milieu. Besonders geeignete Verbindungen der Formel III sind im folgenden aufgeführt:

TABELLE 1

| Subst. Nr. | Verbindung |
|---|---|
| 1.1 | $CH_3 - S - CH_2CH_2 - S - CH_2CH_2 - NH_2$ |
| 1.2 | 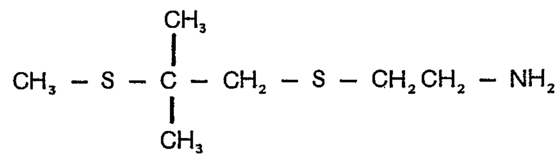 |
| 1.3 | $CH_3 - S - CH_2CH_2 - S - CH_2CH_2CH_2 - NH_2$ |
| 1.4 | $C_2H_5 - S - CH_2CH_2 - S - CH_2CH_2 - NH_2$ |
| 1.5 | 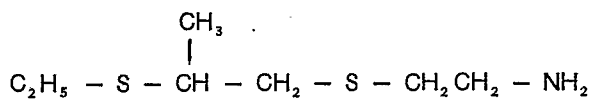 |
| 1.6 | 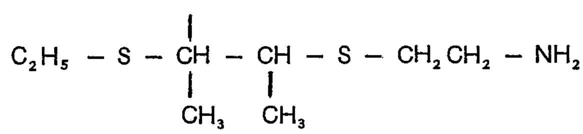 |
| 1.7 | 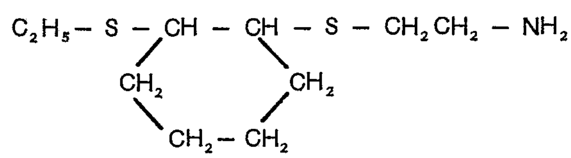 |
| 1.8 | $C_2H_5 - S - CH_2CH_2 - S - CH_2CH_2CH_2 - NH_2$ |
| 1.9 | $CH_3 - (CH_2)_2 - S - CH_2CH_2 - S - CH_2CH_2 - NH_2$ |
| 1.10 | 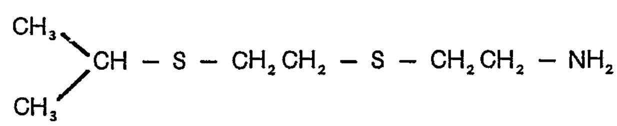 |
| 1.11 | 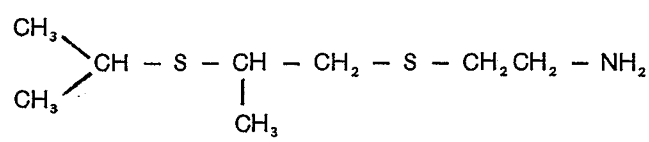 |
| 1.12 | $CH_3 - CH_2CH_2CH_2 - S - CH_2CH_2 - S - CH_2CH_2 - NH_2$ |
| 1.13 | 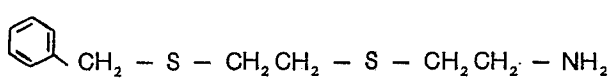 |
| 1.14 | 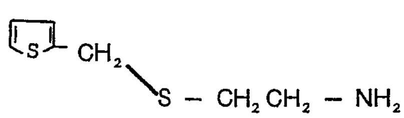 |

Besonders geeignete Isocyanate sind:
Methylisocyanat, Ethylisocyanat, 2-Methoxyethylisocyanat, Methansulfonylisocyanat, bevorzugt in maskierter Form als N-Methansulfonyl-O-ethylurethan, Ethansulfonylisocyanat, Propansulfonylisocyanat, Acetylisocyanat.

Besonders geeignete Dicarbonsäureanhydride sind:
Bernsteinsäureanhydrid, Glutarsäureanhydrid, 2-Ethyl-mercaptobernsteinsäureanhydrid, Maleinsäureanhydrid, Hexahydrophthalsäureanhydrid, 1,2,3,6-Tetrahydrophthalsäureanhydrid, Phthalsäureanhydrid, Pyromellithsäureanhydrid, Naphthalsäureanhydrid, 4-Sulfonaphthalsäureanhydrid, Diglykolsäureanhydrid.

Besonders geeignete Sulfocarbonsäureanhydrid, sind:
3H-2,1-Benzoxathiol-3-1,1-dioxid, (2-Sulfobenzoesäureanhydrid), 3-Sulfopropionsäureanhydrid.

Ganz besonders geeignete Thioether der allgemeinen Formel I sind in den folgenden Tabelle 2 und 3 aufgeführt und entsprechen folgenden allgemeinen Formeln

V $$R^1{-}S{-}CH_2CH_2{-}S{-}CH_2CH_2{-}NH{-}CO{-}R^9$$

VI $$R^1{-}S{-}CR^2HCR^4H{-}S{-}(CH_2)_n{-}NH{-}CO{-}R^9$$

worin $R^1$, $R^2$, $R^4$, $R^9$ und n die unter Formel I und insbesondere unter Formel II angegebene Bedeutung haben.

TABELLE 2

$R^1 - S - CH_2CH_2 - S - CH_2CH_2 - NH - CO - R^9$

| Subst. Nr. | $R^1$ | $R^9$ | Fp (°K) |
|---|---|---|---|
| 2.1 | $-CH_3$ | $-CH_2CH_2-COOH$ | 381–82° |
| 2.2 | $-CH_3$ | $-NH_2$ | 375–76° |
| 2.3 | $-CH_3$ | $-CH=CH$<br>COOH | 348° |
| 2.4 | $-CH_3$ | 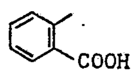 | 391–92° |
| 2.5 | $-CH_3$ | 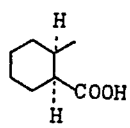 | 379–80° |
| 2.6 | $-C_2H_5$ | $-NH_2$ | 373–74° |
| 2.7 | $-C_2H_5$ | $-NH-SO_2CH_3$ | 377° |
| 2.8 | $-C_2H_5$ | $-CH_2CH_2-COOH$ | 392–93° |
| 2.9 | $-C_2H_5$ | $-CH_2CH_2CH_2-COOH$ | 343° |
| 2.10 | $-C_2H_5$ | $-CH=CH$<br>COOH | 342–43° |
| 2.11 | $-C_2H_5$ | 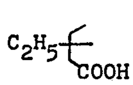 | 331–33° |

| Subst. Nr. | $R^1$ | $R^9$ | Fp (°K) |
|---|---|---|---|
| 2.12 | $-C_2H_5$ | 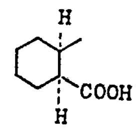 | 367–68° |
| 2.13 | $-C_2H_5$ | 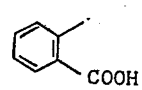 | 391–92° |
| 2.14 | $-C_2H_5$ | 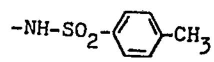 | 327° |
| 2.15 | $-C_2H_5$ | 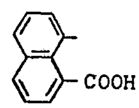 | 406–07° |
| 2.16 | $-C_2H_5$ | $-NHCH_3$ | 348° |
| 2.17 | $CH_3-CH_2-CH_2-$ | $-CH_2CH_2-COOH$ | 396–97° |
| 2.18 | $CH_3-CH_2-CH_2-$ | $-NH_2$ | 356–57° |
| 2.19 | $CH_3-CH_2-CH_2-$ | 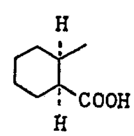 | 340–41° |
| 2.20 | $CH_3-CH_2-CH_2-$ | 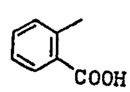 | 382–83° |
| 2.21 | 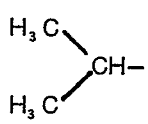 | $-NH_2$ | 369° |
| 2.22 | 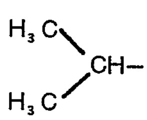 | $-CH_2CH_2-COOH$ | 363° |
| 2.23 | 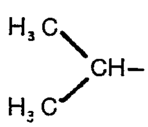 | 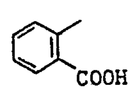 | 387–88° |

| Subst. Nr. | $R^1$ | $R^9$ | Fp (°K) |
|---|---|---|---|
| 2.24 | $H_3C-(CH_2)_3-$ | $-NH_2$ | 356–57° |
| 2.25 | $H_3C-(CH_2)_3-$ | $-CH_2CH_2-COOH$ | 376–77° |
| 2.26 | 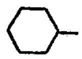 | $-NH_2$ | 383–84° |
| 2.27 | 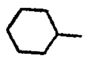 | $-NH-SO_2-CH_3$ | 387–89° |

TABELLE 3

| Subst. Nr. | Formel | Fp (°K) |
|---|---|---|
| 3.1 | $C_2H_5-S-CH(CH_3)-CH_2-S-CH_2-CH_2-NH-CO-$ 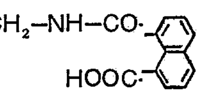 | 390–91° |
| 3.2 | $(CH_3)_2-CH-S-CH(CH_3)-CH_2-S-CH_2-CH_2-NH-CO-CH_2-CH_2-COOH$ | 391–92° |
| 3.3 | 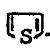 $-CH_2-S-CH_2-CH_2-NH-CO-NH_2$ | 383° |
| 3.4 | 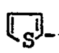 $-CH_2-S-CH_2-CH_2-NH-CO-CH_2-CH_2-COOH$ | 372° |
| 3.5 | $C_2H_5-S-$ 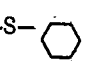 $-S-CH_2-CH_2-NH-CO-CH_2-CH_2-COOH$ | 394–95° |
| 3.6 | $C_2H_5-S-CH_2-CH_2-S-CH_2-CH_2-CH_2-NH-CO-NH_2$ | 343° |
| 3.7 | $C_2H_5-S-CH_2-CH_2-S-CH_2-CH_2-CH_2-NH-CO-CH_2-CH_2-COOH$ | 385° |
| 3.8 | $C_2H_5-S-CH_2-CH_2-S-CH_2-CH_2-CH_2-NH-CO-$ 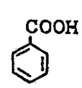 | 403° |
| 3.9 | $C_2H_5-S-CH_2-CH_2-S-CH_2-CH_2-NH-CONH-CO-CH_3$ | |
| 3.10 | $C_2H_5-S-CH_2-CH_2-S-CH_2-CH_2-NH-CONH-CH_2-CH_2-OH$ | |

Nachfolgend wird die Herstellung der Ausgangsamine sowie der erfindungsgemäßen Verbindungen repräsentativ beschrieben:

*Verbindung 1.9: 1-Amino-3,6-dithianonan*

76,4 g (0,64 Mol) 1-Hydroxy-3,thiahexan, hergestellt aus Propan-1-thiol und 2-Chlorethanol in Gegenwart der äquivalenten Menge NaOH, werden mit 50 g Thioharnstoff und 77 g 37%iger Salzsäure 2 Stunden unter Rückfluß erhitzt. Nach dem Erkalten werden zur klaren Lösung des Isothiuroniumsalzes 32 g (0,8 Mol) NaOH in 60 ml Wasser hinzugegeben. Anschließend wird 30 Minuten lang am Dampfbad erhitzt. Das abgeschiedene 1-Mercapto-3-thiahexan wird im Scheidetrichter abgetrennt und in einem Dreihalskolben unter Stickstoff in der Lösung von 68 g (1,7 Mol) NaOH in 130 ml Wasser gelöst. Zu dieser Lösung tropft man bei 333°K insgesamt 74,5 g 2-Chlorethylamin-hydrochlorid in 200 ml Wasser. Man hält nach 1 Stunde am Dampfbad, trennt die abgeschiedene Aminphase mit Ether (300 ml) ab, trocknet mit 40 g KOH-Plätzchen, dampft ein und fraktioniert im Vakuum.

Ausbeute: 96 g (84% d.Th.)
Kp (1,7 mb): 377—378°K
$n_D^{26}$: 1,5272

*Verbindung 1.8: 1-Amino-4,7-dithianonan*

106 g (1 Mol) 1-Hydroxy-3-thiapentan werden mit 76 g (1 Mol) Thioharnstoff und 110 g (1,1 Mol) 37%iger Salzsäure 3 Stunden unter Rückfluß gehalten. Bei 313—323°K wird anschließend die Lösung von 68 g (1,21 Mol) KOH in 100 ml Wasser unter Rühren zugetropft. Man erhitzt 40 Minuten auf 363°K, trennt das abgeschiedene 3-Thiapentan-1-thiol im Scheidetrichter ab und löst es unter $N_2$ bei 303—313°K in einem 1000 ml Dreihalskolben in 400 g 40% KOH (2,85 Mol KOH). Bei 343°K tropft man dann unter Rühren die Lösung von 130 g 3-Chlorpropylamin-hydrochlorid (1 Mol) in 160 ml Wassere zu. Man rührt noch 3 Stunden bei 363°K weiter, kühlt auf 323°K und trennt im Scheidetrichter. Die Ölphase wird zweimal im Vakuum fraktioniert.

Ausbeute: 105 g (58% d.Th.)
$Kp_{(3\ mb)}$: 388—393°K
$n_D^{25}$: 1,5290

*Verbindung 1.7: 1-Amino-4,5-tetramethylen-3,6-dithiaoctan*

Diese Verbindung wird in folgenden Stufen hergestellt:

a) 2-Ethylmercapto-cyclohexanol:
Zu 65 g Ethanthiol (1,05 Mol) in 300 ml Methanol tropft man nach Zugabe von 4 ml DBN (1,5-Diazo-bicyclo-[4,3,0]non-5-en) 98,1 g (1 Mol) Cyclohexenoxid (Aldrich). Man hält im Dampfbad über Nacht unter Rückfluß, wobei die Rückflußtemperatur von 325°K auf 338°K ansteigt. Man stellt mit $CO_2$ auf pH ~ 8, destilliert das Methanol ab und fraktioniert im Vakuum.
Ausbeute: 155 g (97% d.Th.)

b) 2-Ethylmercapto-cyclohexanthiol:
154 g (0,96 Mol) 3-Ethylmercapto-cyclohexanol, 76 g Thioharnstoff (1 Mol) und 150 ml konz. Salzsäure werden 3 Stunden unter Rückfluß gehalten. Die Isothiuroniumsalzlösung wird im Scheidetrichter von öligen Nebenprodukten abgetrennt und im Dampfbad mit 70 g NaOH (1,75 Mol) in 100 ml Wasser 1 Stunde erhitzt.
Zur Neutralisation wird $CO_2$ zugegeben, die Mercaptanphase im Scheidetrichter abgetrennt und im Vakuum fraktioniert.
Ausbeute: 110 g (65% d.Th.)
$Kp_{(3,5\ mb)}$: 371—376°K

c) 1-Amino-4,5-tetramethylen-3,6-dithiaoctan:
110 g (0,625 Mol) 2-Ethylmercapto-cyclohexanthiol werden unter $N_2$ in der Lösung von 107,5 g KOH (1,92 Mol) in 110 ml Wasser aufgenommen. Nach Zugabe von 110 ml Methanol wird bei 328—338°K die Lösung von 74,2 g (0,64 Mol) 2-Chlorethylamin-hydrochlorid in 80 ml Wasser zugetropft. Man hält noch 3 Stunden am Dampfbad, kühlt ab, nimmt die Aminphase in Toluol auf, trocknet über NaCl, vertreibt das Toluol im Vakuum und fraktioniert im Vakuum.
Ausbeute: 106 g (77% d.Th.)
$Kp_{(4,5\ mb)}$: 420—425°K

*Verbindung 1.14: 1-Amino-4-(2-thienyl)-3-thiabutan*

Diese Verbindung wird in folgenden Stufen hergestellt:

a) 2-Thienylmethanthiol:
87 g (0,76 Mol) 2-Thienylmethanol, 58 g (0,76 Mol) Thioharnstoff und 70 ml konz. Salzsäure werden 1 Stunde auf dem Dampfbad erhitzt. Man gibt dann 33 g (0,825 Mol) NaOH in 40 ml Wasser zu, hält 20 Minuten am Dampfbad und trennt im Scheidetrichter.

b) 1-Amino-4(2-thienyl)-3-thiabutan:
Das Rohprodukt aus a) wird unter $N_2$ in der Lösung von 128 g KOH (2,3 Mol) in 150 ml Wasser aufgenommen. Bei 323°K tropft man unter Rühren die Lösung von 88,2 g (0,76 Mol)

2-Chlorethylamin-hydrochlorid in 100 ml Wasser zu. Man hält noch 3 Stunden am Dampfbad, nimmt mit 200 ml Toluol auf, wäscht die Toluolphase mit 200 ml Wasser, trocknet mit NaCl, engt ein und fraktioniert im Vakuum.

Ausbeute 65 g (49% d.Th.)

$Kp_{(8\ mb)}$: 414—425°K

*Verbindung 2.2: N-(3,6-Dithia)heptylharnstoff*

15,1 g (0,1 Mol) 1-Amino-3,6-dithiaheptan werden mit 10%iger Salzsäure gelöst, der pH der Lösung beträgt 4. Zu dieser Lösung gibt man 8,9 g (0,11 Mol) Kaliumcyanat in 20 ml Wasser. Die Lösung erwärmt sich und scheidet ein Öl ab. Man erwärmt 30 Minuten am Dampfbad, läßt erkalten, gießt vom kristallisierten Produkt ab und kristallisiert einmal aus Methanol-Wasser 1:1 und einmal aus Essigester um.

Ausbeute: 15,2 g (78% d.Th.)

Fp: 375—376°K

| | | | | | |
|---|---|---|---|---|---|
| Analyse: | ber. a. | $C_6H_{14}N_2OS_2$ | C: 37.09 | H: 7.26 | S: 33.00 |
| | gef. | | 36.8 | 7.40 | 32.69 |

*Verbindung 2.7: N-(3,6-Dithiaoctyl)-N'-methansulfonylharnstoff*

16,5 g (0,1 Mol) 1-Amino-3,6-dithiaoctan, hergestellt nach den Angaben der DE—OS 1 904 149, und 17 g N-Methansulfonyl-o-ethylurethan, hergestellt nach Cassaday und Ainsworth, J.Org.Chem. 23, 923—926 (1958), werden im Ölbad 2 Stunden auf 403°K gehalten, das abgespaltene Ethanol wird nit $N_2$ ausgeblasen. Nach dem Erkalten wird das kristallisierte Produkt zweimal aus Ethylacetat umkristallisiert.

Ausbeute: 15 g

| | | | | | |
|---|---|---|---|---|---|
| Analyse: | ber. a. | $C_8H_{18}N_2O_3S_3$ | C: 33.55 | H: 6.33 | S: 33,58 |
| | gef. | | 33.8 | 6.5 | 33.6 |

*Verbindung 2.8: N-(3,6-Dithiaoctyl)succinamidsäure*

Zur Lösung von 11,0 g Bernsteinsäureanhydrid in 60 ml Dichlorethan gibt man 16,5 g 1-Amino-3,6-dithiaoctan. Die Temperatur steigt spontan auf 343°K. Nach dem Erkalten im Eisbad saugt man ab und kristallisiert zweimal aus Ethylacetat-Isopropanol 3:1 un.

Ausbeute: 18,6 g (70% d.Th.)

| | | | | | |
|---|---|---|---|---|---|
| Analyse: | ber. a. | $C_{10}H_{19}NO_3S_2$ | C: 45.26 | H: 7.16 | S: 24.26 |
| | gef. | | 45.5 | 7.15 | 24.15 |

*Verbindung 2.16: N-(3,6-Dithiaoctyl)-N'-methyl-Harnstoff*

Zur Lösung von 16,5 g 1-Amino-3,6-dithiaoctan in 40 ml Ethylacetat tropft man bei RT 6 g Methylisocyanat in 15 ml Ethylacetat. Der ausgefallene Harnstoff wird zweimal aus Methanol-Wasser umkristallisiert.

Ausbeute: 12 g (43% d.Th.)

| | | | | | |
|---|---|---|---|---|---|
| Analyse: | ber. a. | $C_8H_{18}N_2OS_2$ | C: 43.21 | H: 8.16 | S: 28.84 |
| | gef. | | 43.0 | 7.95 | 28.70 |

Die erfindungsgemäßen Thioether zeichnen sich durch hohe chemische Stabilität aus und sind leicht und sicher in hoher Reinheit zu gewinnen. Sie sind bei den während der Emulsionsherstellung üblichen Bedingungen so stabil, daß sie selbst bei Verarbeitung bei höheren Temperaturen keinen Schleier verursachen.

Die erfindungsgemäßen Thioether zeichnen sich weiterhin dadurch aus, daß sie schon in sehr geringen Konzentrationen wirksam sind. Bei Durchführung einer Ostwald-Reifung unterstützen sie diese, während sie die Keimzahl bei der üblichen Fällung monodisperser Emulsionen deutlich verringern.

Zur Herstellung von photographischen Silberhalogenidemulsionen durch Fällung von Silberhalogeniden in einem kolloidalen Bindemittelmedium werden die erfindungsgemäßen Thioether vorzugsweise dem Bindemittel oder einem der zur Fällung des Silberhalogenid verwendeten wasserlöslichen Salzes zugesetzt, z.B. dem wasserlöslichen Halogenid, beispielsweise einem Alkalihalogenid. Der Zusatz kann vor Beginn der Fällung erfolgen, spätestens aber während der Fällung.

Die erfindungsgemäßen Thioether können innerhalb weiter pH- und pAg-Bereiche sowie in weiten Temperaturbereichen verwendet werden. Der pH-Wert liegt im allgemeinen zwischen 2 und 9, insbesondere zwischen 3 und 7; der pAg-Wert liegt im allgemeinen oberhalb 6,8, insbesondere zwischen 8 und 10; der temperaturbereich liegt in allgemeinen zwischen 313°K und 358°K, insbesondere zwischen 318°K und 353°K.

Die erfindungsgemäßen Thioether können innerhalb eines großen Konzentrationsbereiches eingesetzt werden. Als besonders vorteilhaft haben sich dabei Konzentrationen von 0,05 bis 50 g, insbesondere von 0,1 bis 25 g Thioether pro Mol Silberhalogenid erweisen.

In einer besonders bevorzugten Ausführungsform werden die erfindungsgemäßen Thioether zusammen mit den aus der deutschen Offenlegungsschrift 2 159 379 bekannten Phosphorsäureamiden der folgenden Formel:

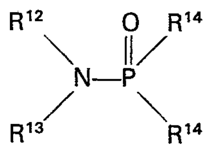

worin bedeuten:

$R^{12}$ Wasserstoff, eine gesättigte oder olefinisch ungesättigte aliphatische Gruppe, Aryl, Cycloalkyl oder Acyl;

$R^{13}$ Wasserstoff, eine gesättigte oder olefinisch ungesättigte aliphatische Gruppe, Aryl oder Cycloalkyl;

$R^{14}$ —O—$R^{15}$ oder 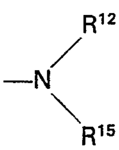

$R^{15}$ eine gesättigte oder olefinisch ungesättigte aliphatische Gruppe, Aryl oder Cycloalkyl;
und/oder mit dem aus der deutschen Offenlegungsschrift 2 015 404 bekannten Kieselsäuresol, das in Mengen bis zu 50 Vol.-% einen wasserlöslichen, kurzkettigen aliphatischen Alkohol und/oder in Mengen bis zu 20 Gew.-%, bezogen auf den Feststoffgehalt des Kieselsäuresols, ein wasserlösliches, organisches Polymeres enthält, verwendet.

In einer weiteren Ausführungsform der Erfindung werden die Thioether entsprechend Formel I auch als Reifzusätze zur chemischen Reifung verwendet. In diesem Fall werden ein hoher pAg-Wert und ein niedriger pH-Wert bevorzugt. Es wird angenommen, daß unter diesen Bedingungen Silberionen gezielt an die Reifzentren herangeführt werden und dadurch ein gezieltes Keimwachstum ermöglichen.

Die erfindungsgemäßen Thioether können für die Herstellung üblicher Silberhalogenidemulsionen verwendet werden. Diese können als Silberhalogenid Silberchlorid, Silberbromid, Silberiodid oder Gemische davon enthalten. Bei den Emulsionen kann es sich um Negativemulsionen oder Direktpositivemulsionen handeln. Die Körner der Emulsionen können einen geschichteten Kornaufbau aufweisen.

Photographisches Material mit in Gegenwart der erfindungsgemäßen Thioether hergestellten Emulsionen kann mit den üblichen Farbentwicklersubstanzen entwickelt werden, z.B.

N,N-Dimethyl-p-phenylendiamin
4-Amino-3-methyl-N-äthyl-N-methoxyäthylanilin
2-Amino-5-diäthylaminotoluol
N-Butyl-N-$\omega$-sulfobutyl-p-phenylendiamin
2-Amino-5-(N-äthyl-N-$\beta$-methansulfonamidäthyl-amino)-toluol
N-Äthyl-N-$\beta$-hydroxyäthyl-p-phenylendiamin
N,N-Bis-($\beta$-hydroxyäthyl)-p-phenylendiamin
2-Amino-5-(N-äthyl-N-$\beta$-hydroxyäthylamino)-toluol.

Weitere brauchbare Farbentwickler sind beispielsweise beschrieben in J.Amer.Chem. Soc. *73,* 3100 (1951).

Photographisches Material mit in Gegenwart der erfindungsgemäßen Thioether hergestellten Emulsionen kann die üblichen Farbkuppler enthalten, die in der Regel den Silberhalogenidschichten selbst einverleibt sind. So enthält die rotempfindliche Schicht beispielsweise einen nicht-diffundierenden Farbkuppler zur Erzeugung des blaugrünen Teilfarbenbildes, in der Regel einen Kuppler vom Phenoloder $\alpha$-Naphtholtyp. Die grünempfindliche Schicht enthält mindestens einen nicht-diffundierenden Farbkuppler zur Erzeugung des purpurnen Teilfarbenbildes, wobei üblicherweise Farbkuppler vom Typ des 5-Pyrazolons oder des Indazolons Verwendung finden. Die blauempfindliche Schichteinheit schließlich enthält mindestens einen nicht-diffundierenden Farbkuppler zur Erzeugung des gelben Teilfarbenbildes, in der Regel einen Farbkuppler mit einer offenkettigen Ketomethylengruppierung. Farbkuppler dieser Art sind in großer Zahl bekannt und in einer Veilzahl von Patentschriften beschrieben.

Beispielhaft sei hier auf die Veröffentlichung "Farbkuppler" von W. PELZ in "Mitteilungen aus den Forschungslaboratorien der Agfa, Leverkusen/München", Band III (1961), und K. VENKATARAMAN in "The Chemistry of Synthetic Dyes", Vol. 4, 341—387, Academic Press, 1971, hingewiesen.

Als weitere nicht-diffundierende Farbkuppler können 2-Äquivalentkuppler verwendet werden;

diese enthalten in der Kupplungsstelle einen abspaltbaren Substituenten, so daß sie zur Farbbildung nur zwei Äquivalente Silberhalogenid benötigen im Unterschied zu den üblichen 4-Äquivalentkupplern. Zu den einsetzbaren 2-Äquivalentkupplern gehören beispielsweise die bekannten DIR-Kuppler, bei denen der abspaltbare Rest nach Reaktion mit Farbentwickleroxidationsprodukten als diffundierender Entwicklungsinhibitor in Freiheit gesetzt wird. Weiterhin können zur Verbesserung der Eigenschaften des photographischen Materials die sogenannten Weißkuppler eingesetzt werden.

Die nicht-diffundierenden Farbkuppler und farbgebenden Verbindungen werden den lichtempfindlichen Silberhalogenidemulsionen oder sonstigen Gießlösungen nach üblichen bekannten Methoden zugesetzt. Wenn es sich um wasser- oder alkalilösliche Verbindungen handelt, können sie den Emulsionen in Form von wässrigen Lösungen, gegebenenfalls unter Zusatz von mit Wasser mischbaren organischen Lösungsmitteln wie Ethanol, Aceton oder Dimethylformamid, zugesetzt werden. Soweit es sich bei den nicht-diffundierenden Farbkupplern und farbgebenden Verbindungen um wasser- bzw. alkaliunlösliche Verbindungen handelt, können sie in bekannter Wiese emulgiert werden, z.B. indem eine Lösung dieser Verbindungen in einem niedrigsiedenden organischen Lösungsmittel direkt mit der Silberhalogenidemulsion oder zunächst mit einer wässrigen Gelatinelösung vermischt wird, worauf das organische Lösungsmittel in üblicher Weise entfernt wird. Ein so erhaltenes Gelatineemulgat der jeweiligen Verbindung wird anschließend mit der Silberhalogenidemulsion vermischt. Gegebenenfalls verwendet man zur Einemulgierung derartiger hydrophober Verbindungen zusätzlich noch sogenannte Kupplerlösungsmittel oder Ölformer; das sind in der Regel höhersiedende organische Verbindungen, die die in den Silberhalogenidemulsion zu emulgierenden, nicht-diffundierenden Farbkuppler und Entwicklungsinhibitor abspaltenden Verbindungen in Form öliger Tröpfchen einschließen. Verwiesen sei in diesem Zusammenhang beispielsweise auf die US-Patentschriften 2 322 027, 2 533 514, 3 689 271, 3 764 336 und 3 765 897.

Als Bindemittel für die photographischen Schichten wird vorzugsweise Gelatine verwendet. Diese kann jedoch ganz oder teilweise durch andere natürliche oder synthetische Bindemittel ersetzt werden. An natürlichen Bindemitteln sind z.B. Alginsäure und deren Derivate wie Salze, Ester oder Amide, Cellulose-Derivate wie Carboxymethylcellulose, Alkylcellulose wie Hydroxyethylcellulose, Stärke oder deren Derivate wie Ether oder Ester oder Caragenate geeignet. An synthetischen Bindemitteln seien erwähnt Polyvinylalkohol, teilweise verseiftes Polyvinylacetat, Polyvinylpyrrolidon.

Die Emulsionen können auch chemisch sensibilisiert werden, z.B. durch Zusatz schwefelhaltiger Verbindungen bei der chemischen Reifung, beispielsweise Allylisothiocyanat, Allylthioharnstoff und Natriumthiosulfat.

Als chemische Sensibilisatoren können ferner auch Reduktionsmittel, z.B. die in den belgischen Patentschriften 493 464 oder 568 687 beschriebenen Zinnverbindungen, ferner Polyamine wie Diethylentriamin oder Aminoethylsulfinsäure-Derivate, z.B. gemäß der belgischen Patentschrift 547 323, verwendet werden.

Geeignet als chemische Sensibilisatoren sind auch Edelmetalle bzw. Edelmetallverbindungen wie Gold, Platin, Palladium, Iridium, Ruthenium oder Rhodium. Diese Methode der chemischen Sensibilisierung ist in dem Artikel von R. KOSLOWSKY, Z.Wiss. Phot. *46*, 65—72, (1951) beschrieben.

Es ist ferner möglich, die Emulsionen mit Polyalkylenoxid-Derivaten zu sensibilisieren, z.B. mit Polyethylenoxid eines Molekulargewichtes zwischen 1.000 und 20.000, ferner mit Kondensationsprodukten von Alkylenoxiden und aliphatischen Alkoholen, Glykolen, cyclischen Dehydratisierungsprodukten von Hexitolen, mit alkylsubstituierten Phenolen, aliphatischen Carbonsäuren, aliphatischen Aminen, aliphatischen Diaminen und Amiden. Die Kondensationsprodukte haben ein Molekulargewicht von mindestens 700, vorzugsweise von mehr als 1000. Zur Erzielung besonderer Effekte kann man diese Sensibilisatoren selbstverständlich kombiniert verwenden, wie in der belgischen Patentschrift 537 278 und in der britischen Patentschrift 727 982 beschrieben.

Die Emulsionen können auch optisch sensibilisiert sein, z.B. mit den üblichen Polymethinfarbstoffen wie Neutrocyanine, basischen oder sauren carbocyaninen, Rhodacyaninen, Hemicyaninen, Styrylfarbstoffen und Oxonolen.

Derartige Sensibilisatoren sind in dem Werk von F. M. HAMER "The Cyanine Dyes and Related Compounds", 1964, Interscience Publishers, John Wiley and Sons, New York, beschrieben.

Die Emulsionen können die üblichen Stabilisatoren enthalten, wie z.B. homöopolare oder salzartige Verbindungen des Quecksilbers mit aromatischen oder heterocyclischen Ringen wie Mercaptotriazole, einfache Quecksilbersalze, Sulfoniumquecksilberdoppelsalze und andere Quecksilberverbindungen. Als Stabilisatoren sind ferner geeignet Azaindene, vorzugsweise Tetra- oder Pentaazaindene, insbesondere solche, die mit Hydroxyl- oder Aminogruppen substituiert sind. Derartige Verbindungen sind in dem Artikel von BIRR, Z.Wiss.Phot. *47*, (1952) 2—58, beschrieben. Weitere Geeignete Stabilisatoren sind u.a. heterocyclische Mercaptoverbindungen, z.B. Phenylmercaptotetrazol, quaternäre Benzthiazol-Derivate und Benzotriazol.

Die Emulsionen können in der üblichen Weise gehärtet sein, beispielsweise mit Formaldehyd oder halogensubstituierten Aldehyden, die eine Carboxylgruppe enthalten wie Mucobromsäure, Diketonen, Methansulfonsäureester und Dialdehyden.

Weiterhin können die photographischen Schichten mit Härtern des Epoxityps, des heterocyclischen Ethylenimins oder des Acryloyltyps gehärtet werden. Beispiele derartiger Härter sind z.B. in der deutschen Offenlegungsschrift 2 263 602 oder in der britischen Patentschrift 1 226 655 beschrieben. Weiterhin ist es auch möglich, die Schichten gemäß dem Verfahren der deutschen Offenlegungsschrift 2 218 009 zu härten, um farbphotographische Materialien zu erzielen, die für eine Hochtemperaturverarbeitung geeignet sind.

Es ist ferner möglich, die photographischen Schichten bzw. die farbphotographischen Mehrschichtenmaterialien mit Härtern der Daizin-, Triazin- oder 1,2-Dihydrochinolin-Reihe zu härten, wie in den britischen Patentschriften 1 193 290, 1 251 091, 1 306 544, 1 266 655, der französischen Patentschrift 71 02 716 oder der deutschen Patentanmeldung P 23 32 317.3 (A—G 1110) beschrieben ist. Beispiele derartiger Härter sind alkyl- oder arylsulfonylgruppenhaltige Diazin-Derivate, Derivate von hydrierten Diazinen oder Triazinen, wie z.B. 1,3,5-Hexhydrotriazin, fluorsubstituierte Diazin-Derivate, wie z.B. Fluorpyrimidin, Ester von 2-substituierten 1,2-Dihydrochinolin- oder 1,2-Dihydroisochinolin-N-carbonsäuren. Brauchbar sind weiterhin Vinyl sulfonsäurehärter, Carbodiimid- oder Carbamoylhärter, wie z.B. in den deutschen Offenlegungsschriften 2 263 602, 2 225 230 und 1 808 685, der französischen Patentschrift 1 491 807, der deutschen Patentschrift 872 153 und der DDR-Patentschrift 7218 beschrieben. Weitere brauchbare Härter sind beispielsweise in der britischen Patentschrift 1 268 550 beschrieben.

Die vorliegende Erfindung kann für das sogenannte Silberfarbbleichverfahren eingesetzt werden. Weiterhin ist die vorliegende Erfindung auch für das Color-Sofortbild-Verfahren bzw. Farbübertragungsverfahren geeignet. Bei diesen Verfahren diffundieren die Farbstoffe für die Teilfarbbilder in eine Bildempfangsschicht, wo sie fest verankert werden oder die Farbkuppler diffundieren in die Bildempfangsschicht, wo sie nach üblicher farbgebender Entwicklung zu dem Bildfarbstoff umgesetzt werden.

Das lichtempfindliche Material enthält dabei im allgemeinen drei lichtempfindliche Emulsionsschichten, wobei jeder dieser Schichten ein farbgebendes System zugeordnet ist. Unter farbgebendem System wird dabei eine in der jeweiligen Schicht diffusionsfest eingelagerte Verbindung verstanden, die einen Farbstoff oder ein Farbstoffvorprodukt darstellt und die bei der Entwicklung in Gegenwart der alkalischen Verarbeitungsmasse unter der Einwirkung von bildmäßig entstehenden Oxidationsprodukten von photographischen Entwicklern diffundierende, vorzugsweise Säuregruppen enthaltende Farbstoffe abspaltet. Hierfür stehen die verschiedensten chemischen Verbindungen zur Verfügung. Besonders geeignet sind beispielsweise diffusionsfeste farbgebende Substanzen gemäß der US-Patentschrift 3 628 952. Diese Verbindungen spalten bei der Reaktion mit Oxidationsprodukten von Schwarz-Weiß- oder Farbentwicklern diffusionsfähige Farbstoffe ab. Eine weitere brauchbare Verbindungsklasse ist in der deutschen Patentschrift 1 095 115 beschrieben. Die hier genannten Verbindungen ergeben bei der Reaktion mit oxidiertem Farbentwickler diffusionsfähige Farbstoffe, die im allgemeinen zur Klasse der Azomethinfarbstoffe gehören. Ein weiteres brauchbares farbgebendes System ist in den US-Patentschriften 3 443 939 und 3 443 940 beschrieben. Bei diesem System werden unter der Einwirkung von oxidierten Entwicklersubstanzen unter Ringschlußbildung diffusionsfähige Farbstoffe abgespalten.

Farbübertragungsverfahren und in solchen Verfahren verwendete Kuppler, die im Rahmen der vorliegenden Erfindung angewendet werden können, sind ferner in den US-Patentschriften 2 983 606, 3 087 817, 3 185 567, 3 227 550, 3 227 551, 3 227 552, 3 227 554, 3 253 915, 3 415 644, 3 415 645 und 3 415 646 beschrieben.

Die für solche Color-Sofortbild-Verfahren verwendeten lichtempfindlichen Materialien besitzen im allgemeinen folgenden Aufbau:

Blauempfindliche Silberhalogenidemulsionsschicht;
Schicht mit Gelbfarbstoff abgebendem System;
Trennschicht;
grünsensibilisierte Silberhalogenidemulsionsschicht;
Schicht mit Purpurfarbstoff abgebendem System;
Trennschicht;
rotsensibilisierte Silberhalogenidemulsionsschicht;
Schicht mit Blaugrünfarbstoff abgebendem System.

Die folgenden Beispiele dienen zur Erläuterung.

Beispiel 1

Als Vergleichsemulsion wird eine Silberbromidiodid-Emulsion hergestellt mit 5 Mol-% AgJ, wie sie in der Veröffentlichung von TRIVELLI und SMITH in "The Photographic Journal", Band 79, Mai 1939, Seiten 330—338, beschrieben ist. Dabei wird bei 343°K 1/3 der Silbernitratlösung innerhalb einer Minute zu der Halogenidmischung (10% Überschuß am Ende der Fällung) zugeführt und nach einer Pause von 10 Minuten läuft der Rest innerhalb von 20 Minuten zu. Nach der Fällung wird die Emulsion mit Polystyrolsulfonsäure und Herabsetzen des pH mit Mineralsäure auf 3,0 geflockt, dekantiert und gewaschen, um die überschüssigen, wasserlöslichen Salze herauszulösen.

Anschließend wird bei pH 7,0 redispergiert, die erforderliche Gelatinemenge hinzugefügt, mit Natriumthiosulfat und Goldchlorid versehen und bei einer Temperatur zwischen 323°K und 333°K und

einem pH 6,0 und pAg = 8,6 bis 9,2 auf maximale Empfindlichkeit gereift.

Die Vergleichsemulsion wird pro kg versehen mit 10 ml 5%iger Saponinlösung in Wasser, 10 ml 10%igem Formaldehyd in Wasser und 20 ml einer 1%igen methanolischen Lösung von Tetraazainden und auf einen Celluloseacetatträger vergossen. Das Material wird in einem üblichen Sensitometer hinter einem Stufenkeil belichtet und bei 293°K 7 bzw. 16 Minuten lang in folgendem Entwickler entwickelt:

| | |
|---|---|
| Natriumsulfit, sicc. | 70,0 g |
| Borax | 7,0 g |
| Hydrochinon | 3,5 g |
| p-Monomethylaminophenolsulfat | 3,5 g |
| Natriumcitrat | 7,0 g |
| Kaliumbromid | 0,4 g |
| mit Wasser aufgefüllt auf 1 l | |

In weiteren Versuchsreihen werden photographische Emulsionen ebenso hergestellt und verarbeitet mit der Abänderung, daß die aus Tabelle 4 ersichtlichen Mengen an erfindungsgemäßen Thioethern zur bei der Fällung vorgelegten Gelatin Halogenid-Lösung gegeben werden. Die Mengenangaben beziehen sich dabei auf die insgesamt eingesetzte Menge an Silbernitrat.

TABELLE 4

| Subst. | Thioether g /Mol $AgNO_3$ | Mittlerer Korndurchmesser ($\mu$) | Entwicklung | | | | | |
|---|---|---|---|---|---|---|---|---|
| | | | 7 Minuten | | | 16 Minuten | | |
| | | | E | $\gamma$ | S | E | $\gamma$ | S |
| ohne (Typ) | – | 2,3 | 100 | 0,95 | 0,24 | 150 | 1,00 | 0,29 |
| 2.5 | 2,8 | 1,9 | 75 | 1,20 | 0,17 | 175 | 1,60 | 0,21 |
| 2.17 | 2,8 | 2,0 | 60 | 1,10 | 0,18 | 150 | 1,30 | 0,27 |
| 2.18 | 2,8 | 1,6 | 50 | 1,10 | 0,14 | 50 | 1,20 | 0,15 |
| 2.19 | 2,8 | 1,9 | 50 | 1,00 | 0,18 | 75 | 1,30 | 0,23 |
| 1,8-Dihydroxy-3,6-dithiaoctan | 2,8 | 1,7 | nach dem Redispergieren völlig verschleiert | | | | | |

E = rel. Empfindlichkeit

$\gamma$ = Gradation

S = Schleier

Tabelle 4 zeigt, daß bei Verwendung der erfindungsgemäßen Thioether der Schleier reduziert wird und bei relativ kleinen Kristallen noch sehr hohe Empfindlichkeiten gegenüber dem Typ beibehalten werden.

Tabelle 4 zeigt weiterhin, daß das aus der deutschen Offenlegungschrift 1 904 148 bekannte 1,8-Dihydroxy-3,6-dithiaoctan zu einer völligen Verschleierung des photographischen Materials führt.

Beispiel 2

Zur Herstellung einer Silberbromidiodidgelatine-Emulsion werden folgende Lösungen bereitet:

| | | | |
|---|---|---|---|
| Lösung A) | 1000 | ml Wasser | |
| | 10 | g Gelatine | |
| | 30 | g KBr | |
| | 2 | g KJ | Temperatur 323°K |
| Lösung B) | 1000 | ml Wasser | |
| | 40 | g $AgNO_3$ | Temperatur 318°K |

Man gießt Lösung B) innerhalb 5 Minuten gleichmäßig in Lösung A), digeriert anschließend 30 Minuten bei 323°K und kühlt dann auf 293°K ab, gibt 10 ml einer 10%igen wässrigen Polystyrolsulfonsäurelösung dazu und erniedrigt mit Schwefelsäure (25%) den pH auf 3,0, wodurch die Emulsion ausflockt.

Man läßt absitzen und gließt die uberstehende Lösung ab. Zur chemischen Reifung wird das Flockulat in 2000 ml einer 10%igen wässrigen Gelatinelösung (pH 7,5) bei 313°K gelöst.

Nach Auflösen des Flockulates wird auf pH 6,5 eingestellt und eine entsprechende Menge an Schwefelreifer und Goldsalzen hinzugefügt und bei 328°K bis auf volle Empfindlichkeit gereift. Die Emulsion wird pro kg mit 10 ml einer 5%igen wässrigen Lösung von Saponin (Netzmittel), 10 ml einer 5%igen wässrigen Lösung von Formaldehyd (Härtungsmittel) und 20 ml einer 1%igen methanolischen Lösung von 4-Hydroxy-6-methyl-1,3,3a,7-tetraazainden (Stabilisator) versetzt und auf einen Celluloseacetatschichtträger vergossen.

Weitere Emulsionen werden ebenso hergestellt mit der Abänderung, daß zu Lösung A) die aus Tabelle 5 ersichtlichen Mengen an erfindungsgemäßen Thioethern zugegeben werden. Die in Tabelle 5 angegebenen Mengen an Thioethern beziehen sich wiederum auf die Gesamtmenge des eingesetzten Silbernitrates.

Die Belichtung und weitere Verarbeitung dieser Materialien erfolgt wie in Beispiel 1 angegeben und führt zu den in Tabelle 5 angegebenen Werten.

TABELLE 5

| Subst. | Thioether g/Mol $AgNO_3$ | Mittlerer Korndurchmesser ($\mu$) | Entwicklung | | | | | |
|---|---|---|---|---|---|---|---|---|
| | | | 7 Minuten | | | 16 Minuten | | |
| | | | E | $\gamma$ | S | E | $\gamma$ | S |
| ohne (Typ) | – | 0,50 | 100 | 1,10 | 0,18 | 130 | 1,25 | 0,23 |
| 2,5 | 2,8 | 0,25 | 175 | 1,10 | 0,05 | 200 | 1,25 | 0,08 |
| 2,17 | 2,8 | 0,35 | 200 | 1,20 | 0,07 | 230 | 1,50 | 0,15 |
| 2,19 | 2,8 | 0,30 | 175 | 0,70 | 0,05 | 175 | 1,00 | 0,23 |
| 2,18 | 2,8 | 0,40 | 130 | 0,80 | 0,14 | 150 | 0,95 | 0,23 |

E = rel. Empfindlichkeit

$\gamma$ = Gradation

S = Schleier

Beispiel 3

In diesem Beispiel wird die vorteilhafte Verwendung der erfindungsgemäßen Thioether bei der Herstellung monodisperser Emulsionen gezeigt.

Zur Herstellung einer monodispersen Silberbromidemulsion wurden gleichzeitig eine wässrige Lösung von Kaliumbromid sowie eine wässrige Lösung von Silbernitrat bei einer Temperatur von 343°K innerhalb von 32 Minuten bei einem pAg-Wert von 9,6 und einem pH-Wert von 4 nach dem Doppelstrahlverfahren zu einer vorgelegten Gelatinelösung gegeben. In der Verfahrensvariante A) waren in der vorgelegten Gelatinelösung pro Mol zugesetztem Silbernitrat 1,7 g der Verbindung 2.8 enthalten. Gemäß Verfahrensvariante B) waren anstelle der Verbindung 2.8 pro Mol zugesetztem Silbernitrat 1 g 1,8-Dihydroxy-3,6-dithiaoctan enthalten.

Die nach Verfahrensvariante A) und B) erhaltenen Emulsionen wurden analog wie folgt weiterverarbeitet:

Die Emulsionen werden durch Zusatz von 3,3 mg $KAuCl_4$, 80 mg $NH_4SCN$ und 1000 mg $Na_2S_2O_3$ pro Mol Silberhalogenid bei 343°K 30 Minuten lang chemisch gereift.

Anschließend wird bei einem pAg-Wert von 9,9 nach dem Doppelstrahlverfahren weiteres Silberbromid auf die erhaltenen gereiften Emulsionskristalle aufgefällt. Die Dauer der Auffällung betrug 52 Minuten, der mittlere Korndurchmesser der erhaltenen monodispersen Emulsion 0,9$\mu$. Die Emulsion wurde wie üblich geflockt, gewaschen und in einer 10%igen Gelatinelösung redispergiert und 140 Minuten lang einer weiteren chemischen Reifung in Gegenwart von 1,5 mg $KAuCl_4$, 20 mg $NH_4SCN$ und 1000 mg $Na_2S_2O_3$ pro Mol Silbernitrat bei einem pH-Wert von 6,0 gereift.

Die nach Verfahrensvariante A) und B) erhaltenen Emulsionen wurden zusammen mit den im folgenden angegebenen Schichten separat auf einen transparenten Schichtträger aus Polyesterfolie aufgetragen. Die Reihenfolge der einzelnen Schichten ist im folgenden angegeben:

1. Eine Beizschicht aus Gelatine (5,0 g/m²) und 5,7 g pro m² eines Polyurethans aus 4,4-Diphenylmethan-diisocyanat, N-Ethyl-diethanolamin und Epichlorhydrin gemäß der deutschen Patentanmeldung P 26 31 529.9.
2. Eine weiße Pigmentschicht aus Titandioxid (24 g/m²) und Gelatine (2,4 g/m²).
3. Eine schwarze Pigmentschicht aus Ruß (1,9 g/m²) und Gelatine (2,0 g/m²).
4. Eine Farbstoffschicht, enthaltend 0,9 g/m² folgender Verbindung:

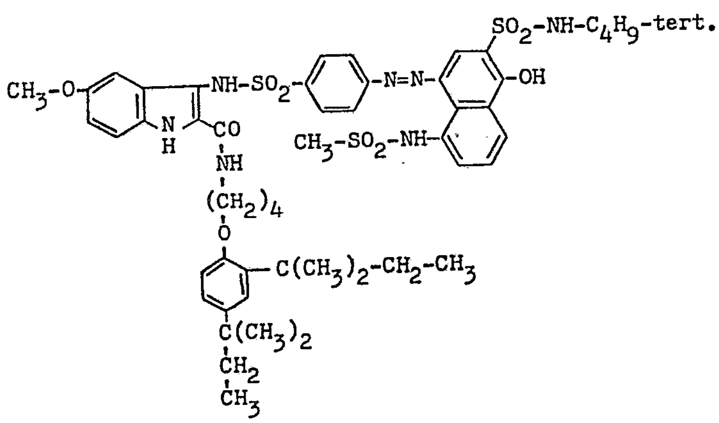

emulgiert in Trikresylphosphat, und Gelatine (1,0 g/m²).

5. Eine Emulsionsschicht mit einer Emulsion gemäß der obenangegebenen Variante A) oder B); die Emulsion ist grünsensibilisiert. Der Gelatineauftrag beträgt 1,4 g/m². Pro Mol Silberhalogenid sind in der Emulsionsschicht 0,066 g Octadecyl-hydrochinonsulfonsäure und 3,4 mg der aus der deutschen Patentanmeldung P 27 46 965.4 bekannten Verbindung Nr. 32 enthalten.
6. Eine Schicht aus Octadecyl-hydrochinonsulfosäure (1 g/m²) und Gelatine (1 g/m²).

Jeweils ein Blatt der so hergestellten lichtempfindlichen Materialien wurde durch einen Graukeil belichtet, anschließend mit einem Pastenbehälter versehen, der mit einer Entwicklerpaste versehen war, und über zwei seitlich angebrachte Abstandstreifen von 140$\mu$ Dicke schichtseitig mit einem Abdeckblatt aus Polyesterfolie abgedeckt. Die Entwicklerpaste hatte folgende Zusammensetzung:

| | |
|---|---|
| Kaliumhydroxid | 40 g |
| Benzylalkohol | 1,5 ml |
| p-Formaldehyd | 1 g |
| Ascorbinsäure | 1 g |

| | |
|---|---|
| 5-Methylbenzotriazol | 3 g |
| 1-Phenyl-4-methyl-4-hydroxymethyl-3-pyrazolidon | 2 g |
| Hydroxyethylcellulose | 35 g |

mit Wasser auf 1000 ml aufgefüllt

Der sandwich-artige Bildset wurde durch ein Quetschwalzen paar geführt, wobei sich die Entwicklerpaste zwischen lichtempfindlichen Teil und Abdeckblatt verteilte. Nach einer Entwicklungszeit von 10 Minuten wurde das Abdeckblatt abgetrennt, das Bildelement mit der anhaftenden Paste neutralisiert und die noch verbliebene Paste abgewaschen.

Es wurde ein purpurfarbiges positives Bild erhalten, wobei bei Einsatz der erfindungsgemäßen Thioether (Variante A) weitaus bessere sensitometrische Ergebnisse erhalten werden als bei Verfahrensvariante B):

TABELLE 6

| Variante | Substanz | g $AgNO_3/m^2$ | $D_{max}$ | $D_{min}$ | E | Q |
|---|---|---|---|---|---|---|
| A | 2,8 | 2,05 | 1,73 | 0,23 | 165 | 0,84 |
| B | 1,8-Dihydroxy-3,6-dithiaoctan | 2,01 | 1,38 | 0,24 | 100 | 0,69 |

Q = Quotient aus $D_{max}$ und Silberauftrag

**Patentansprüche**

1. Verfahren zur Herstellung photographischer Materialien mit wenigstens einer Silberhalogenidemulsionsschicht, dadurch gekennzeichnet, daß die Silberhalogenidemulsion in Gegenwart wenigstens eines Thioethers folgender Formel hergestellt wird:

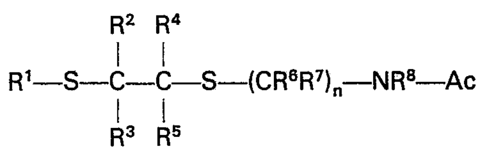

worin bedeuten:

$R^1$ aliphatischer, cycloaliphatischer, Aryl- oder Aralkyl-Rest;
$R^2$, $R^3$, $R^4$, $R^5$, $R^6$, $R^7$ gleich oder verschieden, Wasserstoff oder Alkyl;
$R^8$ Wasserstoff;
Ac Acylrest;
n eine ganze Zahl, mindestens 1.

2. Verfahren nach Anspruch 1, dadurch gekennzeichnet, daß die Fällung der Silberhalogenidkörner der Silberhalogenidemulsion in Gegenwart des Thioethers durch geführt wird.

3. Verfahren nach Anspruch 1, dadurch gekennzeichnet, daß die chemische Reifung der Silberhalogenidkörner der Silberhalogenidemulsion in Gegenwart des Thioethers durchgeführt wird.

4. Verfahren nach Anspruch 2, dadurch gekennzeichnet daß die Fällung der Silberhalogenidkörner zusätzlich in Gegenwart wenigstens einer der folgenden Verbindungen durchgeführt wird:

a) eines Kieselsäuresols, das in Mengen bis zu 50 Vol-% einen wasserlöslichen, kurzkettigen aliphatischen Alkohol und/oder in Mengen bis zu 20 Gew.-% bezogen auf den Feststoffgehalt des Kieselsäuresols, ein wasserlösliches, organisches Polymeres enthält oder

b) eines Phosphorsäureamids der folgenden Formel:

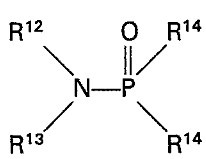

worin bedeuten:

$R^{12}$ Wasserstoff, eine gesättigte oder olefinisch ungesättigte aliphatische Gruppe, Aryl, Cycloalkyl oder Acyl;

$R^{13}$ Wasserstoff, eine gesättigte oder olefinisch ungesättigte aliphatische Gruppe, Aryl oder Cycloalkyl;

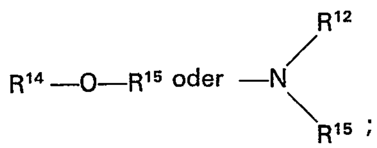

$R^{15}$ eine gesättigte oder olefinisch ungesättigte aliphatische Gruppe, Aryl oder Cycloalkyl.

5. Verfahren nach Anspruch 1, dadurch gekennzeichnet, daß die Thioether folgender Formel entsprechen:

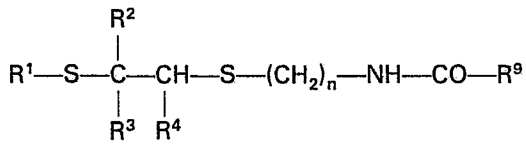

worin bedeuten:

$R^1$ aliphatischer oder cycloaliphatischer Rest mit maximal 6 C-Atomen oder Aralkyl mit maximal 8 C-Atomen;

$R^2$, $R^3$, $R^4$ gleich oder verschieden, Wasserstoff, Alkyl mit 1—7 C-Atomen,

n 2 oder 3;

$R^9$ —NH—$R^{10}$, —$R^{11}$—COOM oder —$R^{11}$—$SO_3$—$M^+$;

M Metallion,

$R^{10}$ Wasserstoff, Alkyl mit 1—3 C-Atomen, Alkoxy-alkyl-Rest oder Alkylsulfonamido-Rest;

$R^{11}$ ein gegebenenfalls zusätzlich substituierter aliphatischer, cycloaliphatischer oder aromatischer Rest,

und/oder

$R^1$ zusammen mit $R^2$ und gegebenenfalls $R^3$ den zur Vervollständigung eines heterocyclischen Ringes erforderlichen Rest

und/oder

$R^4$ zusammen mit $R^2$ oder $R^3$ den zur Vervollständigung eines cycloaliphatischen, insbesondere eines Cyclohexanringes erforderlichen Rest.

6. Photographisches Material mit wenigstens einer Silberhalogenidemulsionsschicht, das gekennzeichnet ist durch den Gehalt an einem Thioether der folgenden Formel:

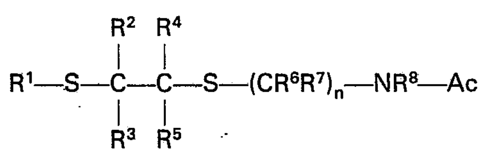

worin bedeuten:

$R^1$ aliphatischer, cycloaliphatischer, Aryl-oder Aralkyl-Rest;

$R^2$, $R^3$, $R^4$, $R^5$, $R^6$, $R^7$ gleich oder verschieden, Wasserstoff oder Alkyl;

$R^8$ Wasserstoff;

Ac Acylrest;

n eine ganze Zahl, mindestens 1.

7. Thioether der folgenden Formel:

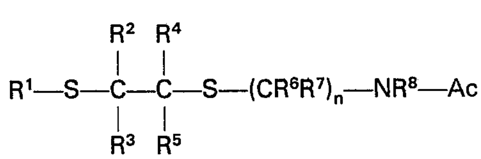

worin bedeuten:

$R^1$ aliphatischer cycloaliphatischer, Aryl- oder Aralkyl-Rest;
$R^2$, $R^3$, $R^4$, $R^5$, $R^6$, $R^7$ gleich oder verschieden, Wasserstoff oder Alkyl;
$R^8$ Wasserstoff;
Ac Acylrest;
n eine ganze Zahl, mindestens 1.

8. Verfahren zur Herstellung photographischer Bilder durch bildmäßiges Belichten eines photographischen Materials mit wenigstens einer lichtempfindlichen Silberhalogenidemulsionsschicht, Entwicklung und übliche Weiterverarbeitung, dadurch gekennzeichnet, daß die Silberhalogenidkörner der Silberhalogenidemulsionsschicht in Gegenwart eines Thioethers folgender Formel hergestellt werden:

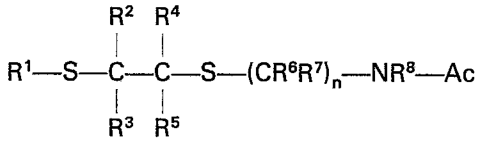

worin bedeuten:
$R^1$ aliphatischer, cycloaliphatischer, Aryl- oder Aralkyl-Rest;
$R^2$, $R^3$, $R^4$, $R^5$, $R^6$, $R^7$ gleich oder verschieden, Wasserstoff oder Alkyl;
$R^8$ Wasserstoff;
Ac Acylrest;
n eine ganze Zahl, mindestens 1.

**Revendications**

1. Procédé pour la préparation de matériaux photographiques comportant au moins une couche d'émulsion d'halogénure d'argent, caractérisé en ce que l'émulsion d'halogénure d'argent est préparée en présence d'au moins un thioéther de formule suivante:

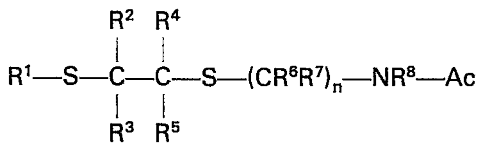

dans laquelle $R^1$ représente un reste aliphatique, cycloaliphatique, aryle ou arylalkyle; $R^2$, $R^3$, $R^4$, $R^5$, $R^6$, $R^7$ sont identiques ou différents et représentent chacun l'hydrogène ou un alkyl; $R^8$ représente l'hydrogène; Ac représente un reste acyle; n est un nombre entier au moins égal à 1.

2. Procédé selon la revendication 1, caractérisé en ce que la précipitation des grains d'halogénure d'argent de l'émulsion d'halogenure d'argent s'effectue en présence du thioéther.

3. Procédé selon la revendication 1, caractérisé en ce que le mûrissage chimique des grains d'halogénure d'argent s'effectue en présence du thioéther.

4. Procédé selon la revendication 2, caractérisé en ce que la précipitation des grains d'halogénure d'argent s'effectue en outre en présence d'au moins un des composés suivants:

(a) un sol d'acide silicique qui contient, en quantités allant jusqu'à 50% en volume, un alcool aliphatique à chaîne courte soluble dans l'eau et/ou, en quantités allant jusqu'à 20% en poids par rapport à la teneur en solides du sol d'acide silicique, un polymère organique soluble dans l'eau, ou

(b) un amide d'acide phosphorique de formule suivante:

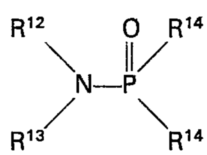

dans laquelle $R^{12}$ représente l'hydrogène, ou un groupe aliphatique saturé ou à insaturation oléfinique, un groupe aryle, cycloalkyle ou acyle; $R^{13}$ représente l'hydrogène ou un groupe aliphatique saturé ou à insaturation oléfinique, aryle ou cycloalkyle;

$R^{14}$ représente un groupe $-O-R^{15}$ ou 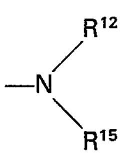

$R^{15}$ est un groupe aliphatique saturé ou à insaturation oléfinique, aryle ou cycloalkyle.

5. Procédé selon la revendication 1, caractérisé en ce que les thioéthers correspondent à la formule suivante:

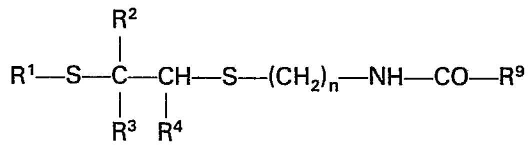

dans laquelle $R^1$ représente un reste aliphatique ou cycloaliphatique jusqu'en $C_6$ ou arylalkyle jusqu'en $C_8$; $R^2$, $R^3$ et $R^4$ sont identiques ou différents et représentent chacun l'hydrogène ou un groupe alkyle en $C_1$—$C_7$; n est égal à 2 ou3; $R^9$ est un groupe —NH—$R^{10}$, —$R^{11}$—COOM ou —$R^{11}$—$SO_3$—$M^+$; M est un ion métallique; $R^{10}$ est un hydrogène ou un groupe alkyle en $C_1$—$C_3$, un reste alcoxyalkyle ou un reste alkylsulfonamido; $R^{11}$ représente un reste aliphatique, cycloaliphatique ou aromatique encore éventuellement substitué et/ou $R^1$ représente avec $R^2$ et éventuellement $R^3$ le reste nécessaire pour compléter un noyau hétérocyclique et/ou $R^4$ représente avec $R^2$ ou $R^3$ le reste nécessaire pour compléter un noyau cycloaliphatique, en particulier un noyau cyclohexane.

6. Matériau photographique comportant au moins une couche d'émulsion d'halogénure d'argent, caractérisé en ce qu'il contient un thioéther de formule suivante:

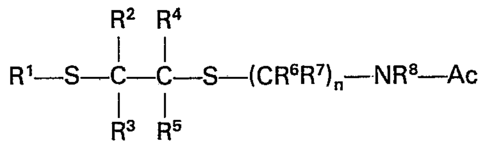

dans laquelle $R^1$ représente un reste aliphatique, cycloaliphatique, aryle ou arylalkyle; $R^2$, $R^3$, $R^4$, $R^5$, $R^6$, $R^7$ sont identiques ou différents et représentent chacun l'hydrogène ou un alkyle; $R^8$ représente l'hydrogène; Ac représente un reste acyle; n est nombre entier au moins égal à 1.

7. Thioéthers de formule suivante:

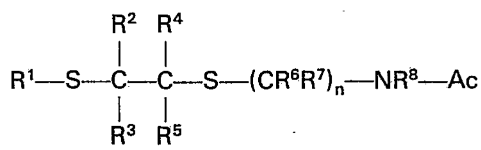

dans laquelle $R^1$ représente un reste aliphatique, cycloaliphatique, aryle ou arylalkyle; $R^2$, $R^3$, $R^4$, $R^5$, $R^6$, $R^7$ sont identiques ou différents et représentent chacun l'hydrogène ou un alkyle; $R^8$ représente l'hydrogène; Ac représente un reste acyle; n est un nombre entier au moins égal à 1.

8. Procédé pour la production d'images photographiques par exposition suivant une image d'un matériau photographique comportant au moins une couche d'émulsion d'halogénure d'argent sensible à la lumière, développement et traitement ultérieurs habituels, caractérisé en ce que les grains d'halogénure d'argent de l'émulsion d'halogénure d'argent sont préparés en présence d'un thioéther de formule suivante:

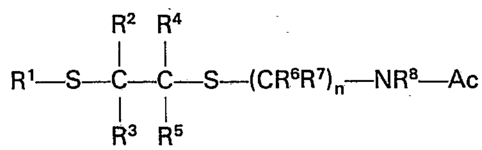

dans laquelle $R^1$ représente un reste aliphatique, cycloaliphatique, aryle ou arylalkyle; $R^2$, $R^3$, $R^4$, $R^5$, $R^6$, $R^7$ sont identiques ou différents et représentent chacun l'hydrogène ou un alkyle; $R^8$ représente l'hydrogène; Ac représente un reste acyle; n est un nombre entier au moins égal à 1.

**Claims**

1. A process for the production of photographic materials containing at least one silver halide emulsion layer, characterised in that the silver halide emulsion is produced in the presence of at least one thioether of the following formula:

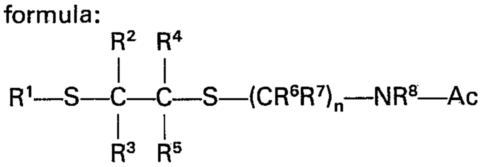

in which

$R^1$ represents an aliphatic or cycloaliphatic or aryl or aralkyl radical;

$R^2$, $R^3$, $R^4$, $R^5$, $R^6$ and $R^7$ are the same or different and represent hydrogen or alkyl;

$R^8$ represents hydrogen;

Ac represents an acyl radical;

$n$ is an integer with a value of at least 1.

2. A process according to Claim 1, characterised in that precipitation of the silver halide grains of the silver halide emulsion is carried out in the presence of the thioether.

3. A process according to Claim 1, characterised in that chemical ripening of the silver halide grains is carried out in the presence of the thioether.

4. A process according to Claim 2, characterised in that precipitation of the silver halide grains is additionally carried out in the presence of at least one of the following compounds:

a) a silica sol which contains a water-soluble short-chain aliphatic alcohol in quantities of up to 50% by volume and/or a water-soluble organic polymer in quantities of up to 20% by weight, based on the solids content of the silica sol, or

b) a phosphoric acid amide of the following formula:

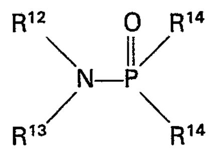

in which:

$R^{12}$ represents hydrogen, a saturated or olefinically unsaturated aliphatic group, aryl, cycloalkyl or acyl;

$R^{13}$ represents hydrogen, a saturated or olefinically unsaturated aliphatic group, aryl or cycloalkyl;

$R^{14}$ represents —O—$R^{15}$ or 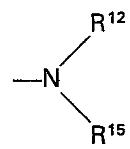

$R^{15}$ represents a saturated or olefinically unsaturated aliphatic group, aryl or cycloalkyl.

5. A process according to Claim 1, characterised in that the thioethers correspond to the following formula:

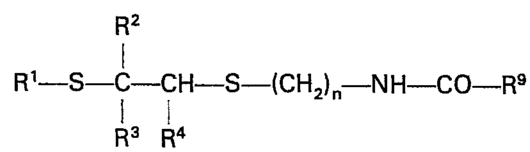

in which:

$R^1$ represents an aliphatic or cycloaliphatic radical containing at most 6 carbon atoms or aralkyl containing at most 8 carbon atoms;

$R^2$, $R^3$ and $R^4$ are the same or different and represent hydrogen, alkyl with 1 to 7 C atoms,

$n$ represents 2 or 3;

$R^9$ represents —NH—$R^{10}$, —$R^{11}$—COOM or —$R^{11}$—$SO_3$—M;

M represents a metal ion,

$R^{10}$ represents hydrogen, alkyl with 1 to 3 C atoms, an alkoxy alkyl radical or an alkyl sulphonamido radical;

$R^{11}$ represents an aliphatic, cycloaliphatic or aromatic radical which may be additionally substituted and/or

$R^1$ together with $R^2$ and optionally $R^3$ represents the radical required to complete a heterocyclic ring, and/or

$R^4$ together with $R^2$ or $R^3$ represents the radical required to complete a cycloaliphatic, in particular a cyclohexane ring.

6. A photographic material containing at least one silver halide emulsion layer, characterised in that it contains a thioether of the following formula:

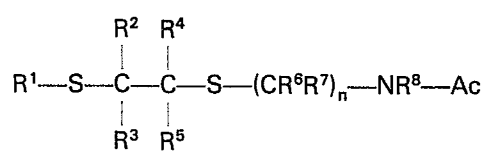

in which:

$R^1$ represents an aliphatic or cycloaliphatic or aryl or aralkyl radical;

$R^2$, $R^3$, $R^4$, $R^5$, $R^6$ and $R^7$ are the same or different and represent hydrogen or alkyl;

$R^8$ represents hydrogen;

Ac represents an acyl radical, and

*n* is an integer with a value of at least 1.

7. Thioethers of the following formula:

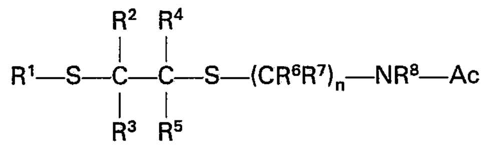

in which:

$R^1$ represents an aliphatic or cycloaliphatic or aryl or aralkyl radical;

$R^2$, $R^3$, $R^4$, $R^5$, $R^6$ and $R^7$ are the same or different and represent hydrogen or alkyl;

$R^8$ represents hydrogen;

Ac represents an acyl radical, and

*n* is an integer with a value of at least 1.

8. A process for the production of photographic images by exposing a photographic material containing at least one photosensitive silver halide emulsion layer to form an image, followed by development and further processing characterised in that the silver halide grains of the silver halide emulsion layer are produced in the presence of a thioether of the following formula:

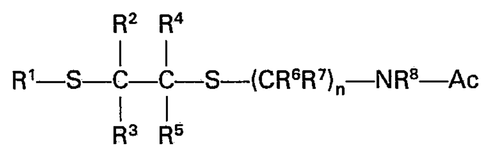

in which:

$R^1$ represents an aliphatic or cycloaliphatic or aryl or aralkyl radical;

$R^2$, $R^3$, $R^4$, $R^5$, $R^6$ and $R^7$ are the same or different and represent hydrogen or alkyl;

$R^8$ represents hydrogen;

Ac represents an acyl radical, and

*n* is an integer with a value of at least 1.